# EUROPEAN PATENT APPLICATION

(11) **EP 1 205 868 A2**
(43) Date of publication of application: **15.05.2002**
(21) Application number: 01204222.2
(22) Date of filing: 02.11.2001
(51) Int. Cl.: G06F 19/00

(54) **A method and apparatus for archiving and distributing enhanced and unenhanced digital medical images**

(30) Priority: 14.11.2000 US 712423
(71) Applicant: EASTMAN KODAK COMPANY, Rochester, New York 14650 (US)
(72) Inventor: Kohm, Kevin S., Eastman Kodak Company, Rochester, New York 14650-2201 (US); Foos, David H., Eastman Kodak Company, Rochester, New York 14650-2201 (US); Van Nostrand, Lance S., Eastman Kodak Company, Rochester, New York 14650-2201 (US)
(74) Representative: Parent, Yves

(57) **Abstract**

A method for distributing digital medical images comprising:
providing an unenhanced digital medical image for storage on an image archive;
providing an image archive for storage of said unenhanced digital medical image;
providing an image server for distribution of said unenhanced digital medical image from said image archive;
providing an image requesting system to request said unenhanced digital medical image stored in said image archive from said image server;
providing a mechanism for said image server to determine the processing capabilities of said image requesting system; such that
if said processing capabilities of said image requesting system include enhancement processing, said image server retrieves said unenhanced digital medical image from said image archive and said image server transmits the said unenhanced digital medical image to said image requesting
system; and
if said processing capabilities of said image requesting system does not include enhancement processing, said image server retrieves said unenhanced digital medical image from said image archive, said image server performs image enhancement on said unenhanced digital medical image to produce an enhanced digital medical image and said image server transmits said enhanced digital medical image to said image requesting system.

## Description

This invention relates in general to digital medical imaging and in particular to the storage and retrieval of images in a digital archive system.

Picture Archiving and Communication Systems (PACS) typically consist of digital acquisition modalities, digital image archives, diagnostic viewing workstations and laser film printers. Digital acquisition modalities include computed radiography (CR), direct digital radiography (DR), computed tomography (CT) and magnetic resonance (MR) among others. The typical workflow is for the image to be acquired at the modality and digital processing performed either on the modality console or on a dedicated quality control workstation prior to transmitting the image to the archive, workstation or printer. In the case of CR and DR in particular, improved image quality and utility can be achieved if the image that is used by the diagnostic workstation does not have the enhancement processing that is performed by the modality applied. However, few workstations can interactively perform this level of processing. In an open systems environment such as DICOM (Digital Imaging Communications in Medicine), there is a need to fulfill high quality images to systems with varying levels of processing capability. Therefore, a need exists to store and transmit either the unenhanced or enhanced images based upon the capability of the device that receives the images.

Current state of the art DICOM medical image archives provide storage of patient images that are ready for transmission and display on approved and validated DICOM display workstations. In a typical scenario, a workstation will query an archive for a list of images that meet certain selection criteria. The archive responds with a list of images, if any, resident on the archive which meet those criteria. The workstation may then request some or all of the available images to be transmitted to the workstation. The archive then retrieves the selected images from storage and transmits them to the workstation. In this scenario, since all images are display-ready, the archive may simply transmit the image data without adjustment to the processing capabilities of the requesting device. The present invention allows the storage and transmission of images that are not display-ready by applying enhancements prior to distribution to a workstation which cannot perform the required processing and transmitting the unenhanced version of the image to workstations capable of performing the enhancement processing.

A recently adopted part of the DICOM standard (Digital Imaging and Communications in Medicine PS 3.3-1999, National Electrical Manufacturers Association, 1999) partially addresses the management of image data which is enhanced or unenhanced. The concept is presented in the DX Series Module by the introduction of Presentation Intent Type. This tag may have the value of FOR PRESENTATION or FOR PROCESSING. If the value of the tag is FOR PRESENTATION, the image has been enhanced such that the image may be displayed without additional processing required. If the value of the tag is FOR PROCESSING, the image must undergo additional enhancement processing prior to display and interpretation. The intent of FOR PROCESING image data is to allow more capable systems which can perform the required enhancement processing to do so while still using a DICOM image object while preserving the capability to provide image data that is display-ready by the use of FOR PRESENTATION. The standard does not however, indicate how these different types of image processing levels should be managed. While several methods are possible, the present invention discloses one method of managing image data that is unenhanced.

U.S. Patent 5,179,651, issued January 12, 1993, inventors Taaffe, et al., describe a methodology whereby an image archive / processor combination can perform enhancement processing on an image prior to transmission to a display workstation if the workstation requests the processing to be performed by the image archive / processor. While this is similar to the present invention, the workstation initiates the request for processing given the complexity of the required processing task and the available workstation resources. In the present invention, the image server determines what processing should be performed prior to transmission. This removes the requirement of the workstation to request the level of processing desired. While this is a possible implementation to achieve a similar result, it imposes additional requirements on the workstation which are removed in the present invention.

U.S. Patent 5,551,428, issued September 3, 1996, inventors Godlewski, et al., describe a system to apply different enhancement parameters to an image based on destination or physician preference. This is related to the present invention in that a database of enhancement parameters is managed by the image source in which the parameters can be varied for each physician. The key difference is that the image data is pushed from the image source to the output device, a film printer. The present invention responds to queries from the destination with the appropriate processing level applied to the image data for the requesting workstation.

U.S. Patent 4,780,821, issued October 25, 1988, inventor Crossley, describes a method for a server computer to determine if a remote computer has the ability to run a requested application or gain access a particular type of data. Again, this is similar to the present invention except that if the server computer does not permit access to the application or data, the remote computer does not receive any data or application to satisfy the request. In the present invention, the image data is not denied if the requesting workstation does not have the ability to process the image data, instead the requested image is first enhanced, allowing the requesting workstation to properly display the image.

According to the present invention, the drawbacks described are eliminated.

According to a feature of the present invention, there is provided a method for distributing digital medical images comprising:
providing an unenhanced digital medical image;
providing an image archive for storage of said unenhanced digital medical image;
providing an image server for distribution of said unenhanced digital medical image from said image archive;
providing an image requesting system to request said unenhanced digital medical image stored in said image archive from said image server;
providing a mechanism for said image server to determine the processing capabilities of said image requesting system; such that
if said processing capabilities of said image requesting system include enhancement processing, said image server retrieves said unenhanced digital medical image from said image archive and said image server transmits the said unenhanced digital medical image to said image requesting system; and
if said processing capabilities of said image requesting system does not include enhancement processing, said image server retrieves said unenhanced digital medical image from said image archive, said image server performs image enhancement on said unenhanced digital medical image to produce an enhanced digital medical image and said image server transmits said enhanced digital medical image to said image requesting system.

The invention has the following advantages over the prior art:
1. This invention enables unenhanced imagery to be transmitted throughout a network without risk of improper display by a less capable system.
2. This invention may be fully implemented in a DICOM network.

Fig. 1 is a block diagram of an image archive / image server system for distribution of enhanced and unenhanced imagery showing the architecture for the acquisition modalities, archive, server and diagnostic workstations.

Fig. 2 is a flow chart for query and retrieval of images which shows the processing steps necessary for requesting and receiving enhanced or unenhanced imagery based on workstation capabilities.

Fig. 3 is a block diagram of an acquisition modality system for distribution of enhanced and unenhanced imagery showing the architecture for an acquisition modality to distribute imagery to workstations or archives.

Fig. 4 is a flow chart for distribution of images from a modality which shows the steps necessary for a modality to send an enhanced or unenhanced image to a destination.

A DICOM network is used in the preferred embodiment of the invention and as such, DICOM terms are used to describe component relationships and transactions. Also, image enhancement is not limited to a single algorithm performed on the image data. Multiple algorithms are typical in medical imaging to enhance an image including, but not limited to, tonescale generation and application, spatial processing, dynamic range adjustment and masking. The multiple individual algorithms should all be considered under the general term image enhancement.

In general, a digital image archive and server are used to manage the storage, processing and transmission of images among multiple modalities and medical imaging workstations. A modality stores images on the archive and a workstation retrieves an image from the image server. In the present invention, the image server acquires the requested image from the archive and performs any necessary processing prior to transmission to the requesting workstation. Consider four types of devices: an imaging modality which produces enhanced imagery, an imaging modality which produces unenhanced or enhanced imagery, a viewing system which can process unenhanced imagery and a viewing system which cannot process unenhanced imagery or alternatively can only display enhanced imagery. For the case of an imaging modality which produces enhanced images only, the enhanced image is transmitted to the archive by the modality. When a workstation requests this image from the image server, the image server retrieves the enhanced image from the archive and immediately transmits the image to the requesting workstation, regardless of the capabilities of the workstation. For the case of an imaging modality which can distribute unenhanced imagery, the unenhanced images are transmitted to the archive by the modality. When a workstation requests this image from the image server, the image server determines if the requesting workstation can properly process the unenhanced image. If the image server determines that the workstation can process the image, the image server retrieves the unenhanced image from the archive and immediately transmits the image to the requesting workstation. If the image server determines that the workstation cannot process the image, the image server first retrieves the unenhanced image from the archive and then performs the enhancement processing on the retrieved image prior to transmitting the image to the requesting workstation. While DICOM does not specify this level of data management, this system can be implemented to be fully DICOM compliant

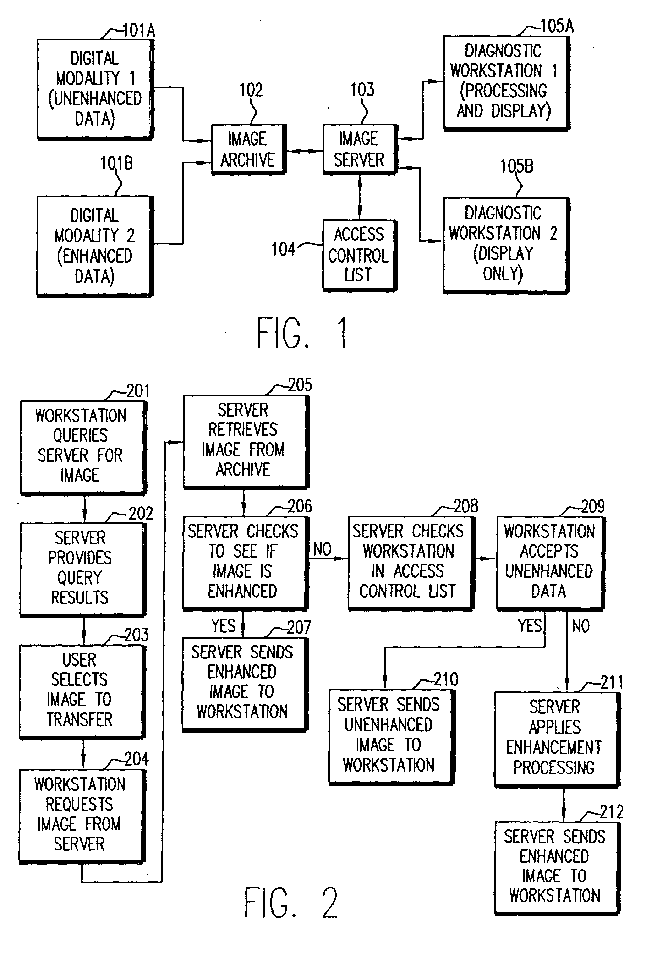

Fig. 1 shows a block diagram of the preferred embodiment of the image archive / image server system according to the present invention. Two digital imaging modalities are shown. Digital modality 1-101A supplies image data that has not been enhanced. Digital modality 2-101B supplies image data that has already been enhanced. Both digital modality 1-101A and digital modality 2-101B transmit acquired images to the image archive 102 for storage. Digital modality 1-101A includes a special DICOM tag in the header, which denotes that the image is unenhanced. This special tag may be an existing element such as "Presentation Intent" in the Direct Radiography (DX) module or the special tag may be a private DICOM element. The image archive 102 is strictly an image storage device, no additional image processing occurs on the image archive 102. The image server 103 works with the image archive 102 such that the image server 103 performs enhancement processing on stored images as needed. The need for the image server 103 to perform enhancement processing is dependent on the capabilities of the requesting device. Each device that can request images from the image server 103 has an entry in the access control list 104. In the preferred embodiment, a field is included in the access control list 104 for each requesting device, which denotes if that particular device can process unenhanced imagery.

Diagnostic workstation 1-105A has the capability to perform enhancement processing on images it receives. When diagnostic workstation 1-105A requests an image from the image server 103, the image server 103 retrieves the image from the image archive 102 and checks the header for the special unenhanced image tag. If the image is enhanced, the image server 103 transmits the image to workstation 1-105A. If the image is unenhanced, the image server 103 checks the access control list 104 and determines that workstation 1-105A can receive unenhanced imagery. The image server 103 then transmits the unenhanced image to workstation 1-105A.

Diagnostic workstation 2-105B does not have the capability to perform enhancement processing on images it receives. When diagnostic workstation 2-105B requests an image from the image server 103, the image server 103 retrieves the image from the image archive 102 and checks the header for the special unenhanced image tag. If the image is enhanced, the image server 103 transmits the image to workstation 2-105B. If the image is unenhanced, the image server 103 checks the access control list 104 and determines that workstation 2-105B cannot receive unenhanced imagery. The image server 103 then applies enhancement processing on the image and transmits the enhanced image to workstation 2-105B. It should be noted that in the last case, since the pixel data has been modified by the image server 103, a new DICOM series unique identifier (UID) must be associated with the image in the DICOM header.

For a more detailed description of the requesting, image processing and transmitting process, refer to Fig. 2. This is a flow chart of typical image request and transmission steps in the preferred embodiment. First, the user of a workstation queries the image server for a list of images 201 based on a set of input criteria. Patient name, accession number and date are typical query parameters. The image server then provides the result of the query 202 to the requesting workstation. The user then selects the image(s) to transfer 203 and the workstation then request the selected image(s) from the image server 204. The image server then retrieves the image(s) from the image archive 205. Next, the image server checks for the special tag in the image header which denotes if the image is enhanced 206. If the tag specifies that the image is enhanced, the image server sends the enhanced image to the workstation 207. If the tag is not present, it is assumed that the image is enhanced. If the tag specifies that the image is unenhanced, the image server then checks the capabilities of the requesting workstation in the access control list 208 and determines if the workstation accepts unenhanced data 209. If the workstation accepts unenhanced data, the image server transmits the unenhanced image to the workstation 210. If the workstation does not accept raw data, the image server applies the enhancement processing 211 and subsequently transmits the enhanced image to the workstation 212.

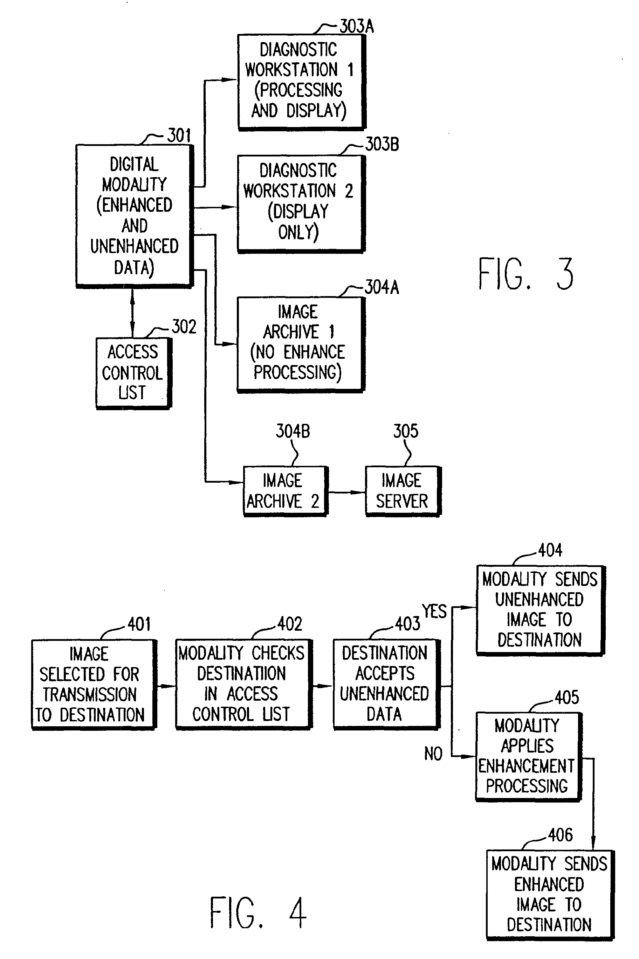

Fig. 3 shows an acquisition modality system for distribution of enhanced and unenhanced images. Digital Modality 301 produces enhanced and unenhanced data. The access control list 302 provides information regarding enhancement capability for each image destination of the digital modality 301. In this example, the image destinations are as follows. Diagnostic Workstation 1-303A has enhancement processing and display capability. Diagnostic Workstation 2-303B has only display capability, or alternatively, no enhancement capability. Image Archive 1-304A has no enhancement processing. Image Archive 2-304B has image enhancement capability via the Image Server 304. The process for determining the image to distribute to each destination is described in Fig. 4. First, an image is selected for transmission to a destination 401. The modality checks destination in the access control list 402. If the destination is determined 403 to accept unenhanced data, the modality sends the unenhanced image to the destination 404. If the destination is determined 403 not to accept unenhanced data, the modality applies enhancement processing to the unenhanced image 405 and sends the enhanced image to the destination 406.

Additional information about the image processing performed may be tracked, although it is not required for implementation. For example, an audit trail may be stored with the enhanced image including the image processing algorithms and parameters, the date and the individual selecting the enhancement. Also, information referencing the original unenhanced image may be kept with the enhanced version to facilitate retrieval of alternate versions. In the same manner, after an image has been enhanced, information may be stored with the unenhanced version indicating the enhanced version exists and where it may be located.

There are alternatives to the preferred embodiment that warrant mention. In the preferred embodiment, the modality appends a special DICOM tag denoting an image is unenhanced. An alternative is for the image server to be programmed such that all images from a given modality are handled as unenhanced. The image server may also be programmed such that all images from a given modality are handled as enhanced. Also in the preferred embodiment, the access control list contains information regarding enhancement processing capability for each workstation. Alternatives to this include having the workstation send a message to the image server stating the ability to process unenhanced imagery. Also, the image server may be directly programmed with information regarding the ability of any workstation to receive unenhanced imagery.

The ability to transmit unenhanced or enhanced imagery based on the capability of the receiving device is not limited to the image server. An acquisition modality itself may act as the image server of the present invention. In this case, when an image is transmitted from the modality, the capabilities of the receiving system are determined from the access control list. The receiving system may be a diagnostic workstation or an image archive.

## Claims

1. A method for distributing digital medical images comprising:
providing an unenhanced digital medical image;
providing an image archive for storage of said unenhanced digital medical image;
providing an image server for distribution of said unenhanced digital medical image from said image archive;
providing an image requesting system to request said unenhanced digital medical image stored in said image archive from said image server;
providing a mechanism for said image server to determine the processing capabilities of said image requesting system; such that
if said processing capabilities of said image requesting system include enhancement processing, said image server retrieves said unenhanced digital medical image from said image archive and said image server transmits the said unenhanced digital medical image to said image requesting system; and
if said processing capabilities of said image requesting system does not include enhancement processing, said image server retrieves said unenhanced digital medical image from said image archive, said image server performs image enhancement on said unenhanced digital medical image to produce an enhanced digital medical image and said image server transmits said enhanced digital medical image to said image requesting system.

2. The method of claim 1 where said processing capabilities are determined by said image server via an access control list.

3. The method of claim 1 where said image enhancement includes a plurality of image enhancement algorithms.

4. The method of claim 1 including storing an audit trail of said image enhancement with said enhanced digital medical image.

5. The method of claim 1 including storing a reference to said unenhanced digital medical image with said enhanced digital medical image.

6. The method of claim 1 including storing a reference to said enhanced digital medical image with said unenhanced digital medical image.

7. The method of claim 1 where said processing capabilities are determined by said image requesting system sending said processing capabilities to said image server.

8. The method of claim 1 where said processing capabilities are determined by said image server such that either all stored images from a single modality are treated as unenhanced or all said stored images from said single modality are treated as enhanced.

9. The method of claim 1 where enhanced imagery is also managed, comprising the additional steps of:
providing an enhanced digital medical image for storage on said image archive; and
if said image requesting system requests said enhanced digital medical image, the said image server retrieves said enhanced digital medical image from said image archive and said image server transmits said enhanced digital medical image to said image requesting system.

10. The method of claim 1 where said image archive and said image server are implemented as a single system.

11. The method of claim 1 whereby the Digital Communications in Medicine (DICOM) standard is used for image transfer and storage.

12. The method of claim 2 whereby the Digital Communications in Medicine (DICOM) standard is used for image transfer and storage.

13. The method of claim 3 whereby the Digital Communications in Medicine (DICOM) standard is used for image transfer and storage.

14. The method of claim 4 whereby the Digital Communications in Medicine (DICOM) standard is used for image transfer and storage.

15. The method of claim 5 whereby the Digital Communications in Medicine (DICOM) standard is used for image transfer and storage.

16. The method of claim 6 whereby the Digital Communications in Medicine (DICOM) standard is used for image transfer and storage.

17. The method of claim 7 whereby the Digital Communications in Medicine (DICOM) standard is used for image transfer and storage.

18. The method of claim 8 whereby the Digital Communications in Medicine (DICOM) standard is used for image transfer and storage.

19. The method of claim 9 whereby the Digital Communications in Medicine (DICOM) standard is used for image transfer and storage.

20. The method of claim 10 whereby the Digital Communications in Medicine (DICOM) standard is used for image transfer and storage.

21. The method for distributing digital medical images comprising:
providing an unenhanced digital medical image on a digital image acquisition modality;
providing a destination image display device;
providing a mechanism for said digital image acquisition modality to determine the processing capabilities of said image display device; such that
if said processing capabilities of said image display device include enhancement processing, said digital image acquisition modality transmits the said unenhanced digital medical image to said image display device; and
if said processing capabilities of said image display device does not include enhancement processing, said digital image acquisition modality performs image enhancement on said unenhanced digital medical image to produce enhanced digital medical image and said digital image acquisition modality transmits said enhanced digital medical image to said image display device.

22. The method of claim 21 where said processing capabilities are determined by said image acquisition modality via an access control list.

23. The method of claim 21 where said image enhancement includes a plurality of image enhancement algorithms.

24. The method of claim 21 including storing an audit trail of said image enhancement with said enhanced digital medical image.

25. The method of claim 21 whereby the Digital Communications in Medicine (DICOM) standard is used for image transfer and storage.

26. The method of claim 22 whereby the Digital Communications in Medicine (DICOM) standard is used for image transfer and storage.

27. The method of claim 23 whereby the Digital Communications in Medicine (DICOM) standard is used for image transfer and storage.

28. The method of claim 24 whereby the Digital Communications in Medicine (DICOM) standard is used for image transfer and storage.

29. The method for distributing digital medical images comprising:
providing an unenhanced digital medical image on a digital image acquisition modality;
providing a destination image archive/server device;
providing a mechanism for said digital image acquisition modality to determine the processing capabilities of said image archive/server device;
if said processing capabilities of said image archive/server device include enhancement processing, said digital image acquisition modality transmits the said unenhanced digital medical image to said image archive/server device; and
if said processing capabilities of said image archive/server device does not include enhancement processing, said digital image acquisition modality performs image enhancement on said unenhanced digital medical image to produce enhanced digital medical image and said digital image acquisition modality transmits said enhanced digital medical image to said image archive/server device.

30. The method of claim 29 where said processing capabilities are determined by said image acquisition modality via an access control list.

31. The method of claim 29 where said image enhancement includes a plurality of image enhancement algorithms.

32. The method of claim 29 including storing an audit trail of said image enhancement with said enhanced digital medical image.

33. The method of claim 29 whereby the Digital Communications in Medicine (DICOM) standard is used for image transfer and storage.

34. The method of claim 30 whereby the Digital Communications in Medicine (DICOM) standard is used for image transfer and storage.

35. The method of claim 31 whereby the Digital Communications in Medicine (DICOM) standard is used for image transfer and storage.

36. The method of claim 32 whereby the Digital Communications in Medicine (DICOM) standard is used for image transfer and storage.

37. A computer storage product software for carrying out the method of claim 1 in a digital computer.

38. A computer storage product software for carrying out the method of claim 21 in a digital computer.

39. A computer storage product storing software for carrying out the method of claim 29 in a digital computer.
